# EUROPEAN PATENT APPLICATION

(11) **EP 3 398 632 A1**
(43) Date of publication of application: **07.11.2018**
(21) Application number: 16881952.2
(22) Date of filing: 10.11.2016
(51) Int. Cl.: A61M 5/20, A61M 5/48, A61M 5/46, A61M 5/158, A61M 37/00

(54) **AUTOMATIC MULTIFUNCTIONAL LIQUID INJECTOR HAVING SEPARATION TYPE OPERATION DEVICE**

(30) Priority: 30.12.2015 KR 20150189188
(71) Applicant: Jskbiomed Inc., Daejeon 34051 (KR)
(72) Inventor: JEON, Jinwoo, Incheon 21397 (KR)
(74) Representative: RatnerPrestia
(86) International application number: PCT/KR2016/012896
(87) International publication number: WO 2017/115999

(57) **Abstract**

The present invention relates to an automatic multifunctional liquid injector having a separation type operation device and enabling: an operation unit to be combined thereto in a replaceable manner such that an operation can be performed by selectively mounting a hydrolifting operation unit and a filler operation unit according to a procedure; and set values to be input, in accordance with each operation, into a control means of a syringe driving unit such that the optimum set value is selected through a user's selection, thereby enabling an effective procedure.

## Description

### Technical Field

The present invention relates to an automatic multifunctional liquid injector having a separable applicator and, more particularly, to an automatic liquid injector which may be selectively mounted with a hydrolifting (hydrolifting injection) applicator unit or a filler applicator unit for a procedure and which has setting values for corresponding operations input into the control means of the syringe actuation unit, so that the optimal setting value may be selected by the user and the procedure may be performed effectively.

### Background Art

As is commonly known, a cosmetic practitioner performs a cosmetic procedure for injecting a filler contained in the barrel of a syringe into the skin of the patient by using, for example, a single-needle syringe.

Also, the cosmetic practitioner performs a cosmetic procedure for injecting a hydrolifting filler contained in the barrel of a syringe into the skin of the patient by using, for example, a multi-needle syringe.

To safely inject various types of liquid, such as a filler, a hydrolifting filler, etc., into the skin of the patient by using the single-needle syringe or the multi-needle syringe, it is crucial that the cosmetic practitioner be knowledgeable about and skilled in the procedure being performed. In particular, the manner in which the liquid is injected into the skin of the patient, such as regards the speed of the injection and the injection amount, etc., not only determine whether or not the cosmetic procedure is successful but also greatly affect the intensity of the pain experienced by the patient.

As such, there has been a need for an automatic liquid injector with which a liquid for a cosmetic procedure may be automatically injected in a consistent manner according to preset values for the speed of the injection, and the injection amount, etc., and which is portable for the convenience of the cosmetic practitioner.

To address these problems, Korean Patent No. 10-1464342 was published as prior art. Such prior art disclosed an apparatus that was manufactured as a small, lightweight device of a linear form, where the practitioner could adjust the depth of the multi-needle inserted into the skin of the patient to a desired value and could make automatic adjustments for the injection time, injection speed, number of injections, amount injected per each injection, etc., according to preset operation modes and setting values.

With the prior art, however, the center of the needle may move depending on the diameter of a syringe, and the user faces a financial burden, since separate apparatuses must be purchased for hydrolifting procedures and filler procedures.

### [Documents of Related Art]

### [Patent Documents]

Korean Patent No. 10-1464342 (Nov. 17, 2014)

### Disclosure

### Technical Problem

The present invention was conceived to resolve the problems described above, and an objective of the present invention is to provide an automatic multifunctional liquid injector that provides economic benefits and work convenience by enabling both hydrolifting procedures and filler procedures with a simple replacement of the applicator unit at the front of a single syringe actuation unit, enabling adjustments to the amount and speed by which a liquid within the syringe is discharged, and having the center of the syringe fixed at a constant position regardless of the diameter of the syringe used with the automatic liquid injector.

The objectives of the embodiments of the present invention are not limited to those described above, and other objectives that are not mentioned herein would be clearly recognizable to the person having ordinary skill in the field of art to which the present invention pertains.

### Technical Solution

In order to accomplish the objectives above, an embodiment of the present invention provides an automatic multifunctional liquid injector having a separable applicator.

The injector may include a syringe actuation unit 100, which includes a syringe 110, a syringe actuation shaft 120 coupled to a rear surface of the syringe 110 and configured to discharge a liquid inside the syringe 110 by way of a piston motion, a syringe actuation means 130 coupled with the syringe actuation shaft 120 to provide an actuation force for the piston motion of the syringe actuation shaft 120, a control means 140 configured to control the syringe actuation means 130, an input means 150 configured to input a command of a user into the control means 140, and a power supply means 160 configured to supply power to the syringe actuation means 130 and the control means 140; and an applicator unit 200 that is coupled in a replaceable manner to a front surface of the syringe actuation unit 100, wherein the applicator unit 200 may perform a hydrolifting procedure or a filler procedure.

The applicator unit 200 may further include a hydrolifting applicator unit 210, which in turn may include a suction tip 211 configured to directly contact a skin surface of the practitioner and suction waste matter, a waste storage chamber 212 configured to store the waste matter suctioned by way of the suction tip 211, and a suction means 213 configured to generate suction pressure at the suction tip 211.

Here, the suction tip 211 may be composed of a suction tip body 211a, a skin contact part 211b coupled with the suction tip body 211a such that the skin contact part 211b may slide into the suction tip body 211a, an elastic element 211c disposed between the suction tip body 211 and the skin contact part 211b to provide an elastic force, and a syringe alignment part 211d that is coupled with the other side of the suction tip body 211a and configured to secure a position of the syringe 110.

Also, the suction tip 211 may further include a syringe position adjustment lever 211e coupled with the syringe alignment part 211d, where the coupling portions of the syringe position adjustment lever 211e and the syringe alignment part 211d may be tapered in a corresponding manner.

In addition, the suction tip 211 may further include a contact operation switch 211f that is formed in corresponding positions of the suction tip body 211a and the skin contact part 211b and is configured to actuate the syringe actuation unit 100 and the suction means 213 by contact.

Furthermore, the suction tip 211 may be formed at an angle of 15 degrees with respect to the skin surface of the person receiving the procedure.

The input means 150 may include a power switch 151, an operation switch 152, a mode change switch 153, and a data edit switch 154.

Here, the input means 150 may be positioned at an upper surface of the syringe actuation unit 100, and the applicator unit 200 may further include a filler applicator unit 220 that includes a syringe protection means 221, of which one side may be coupled to the syringe actuation unit 100 and which may be formed surrounding the syringe 110, and a syringe securing means 222, which may be formed between the syringe protection means 221 and the syringe 110 to secure the syringe 110.

Also, in the syringe protection means 221, a portion of the upper surface may extend to a position above the operation switch 152 of the input means 150, and a portion for contacting the operation switch 152 of the input means 150 may be formed protruding downward.

Furthermore, the syringe actuation unit 100 may include a mode storage device 141, with a plurality of operation modes recorded thereon, where each operation mode may be configured to enter an injection speed and an injection amount that were set beforehand into the control means 140 and stop automatically.

Also, the control means 140 may further include a manual control mode, in which an injection operation may be performed for as long as an operation switch 152 of the input means 150 is pressed.

### Advantageous Effects

An automatic multifunctional liquid injector having a separable applicator according to an embodiment of the present invention may be used for both hydrolifting procedures and filler procedures with a replacement of the applicator unit, so that the financial burden on the cosmetic practitioner resulting from purchasing apparatuses for hydrolifting procedures and filler procedures may be mitigated, and the maintenance of the injector may be made more affordable.

Also, in the past, the center of a syringe would vary depending on the diameter of the inserted syringe, and therefore the position of the inserted needle would be moved in accordance to the movement of the center of the syringe, so that the cosmetic practitioner had to take this into account when performing a procedure. However, with an automatic liquid injector based on the present invention, a syringe rotational securing means and a syringe coupling securing means may be used to keep the syringe secured in a constant position regardless of the diameter of the syringe, so that the cosmetic practitioner may perform a procedure more comfortably and with an improved level of precision.

In addition, information regarding the injection amounts and injection speeds corresponding to various circumstances, including types of procedures such as hydrolifting procedures and filler procedures and types of syringes such as single-needle and multi-needle syringes, may be stored in a mode storage device and loaded for immediate use, for minimizing the impact of the level of skill of the cosmetic practitioner on the outcome of the procedure, and modes such as a manual control mode and a user customized operation mode with which a procedure may be performed according to unique information configured personally by the user may also be included, for a more efficient application to a procedure performed by the cosmetic practitioner.

### Description of Drawings

FIG. 1 shows an automatic multifunctional liquid injector having a separable applicator according to an embodiment of the present invention where a hydrolifting applicator unit is mounted.
FIG. 2 shows a syringe actuation unit according to an embodiment of the present invention.
FIG. 3 is an exploded perspective view of an automatic multifunctional liquid injector having a separable applicator according to an embodiment of the present invention where a hydrolifting applicator unit is mounted.
FIG. 4 is an exploded perspective view of a suction tip according to an embodiment of the present invention.
FIG. 5 shows an example of holding a hydrolifting applicator unit based on an embodiment of the present invention.
FIG. 6 shows an automatic multifunctional liquid injector having a separable applicator according to an embodiment of the present invention where a filler applicator unit is mounted.
FIG. 7 is an exploded perspective view of an automatic multifunctional liquid injector having a separable applicator according to an embodiment of the present invention where a filler applicator unit is mounted.
FIG. 8 shows a syringe protection means 221 according to an embodiment of the present invention.
FIG. 9 shows an example of holding a filler applicator unit based on an embodiment of the present invention.

### Best Mode

The present invention is described below in further detail with reference to the appended drawings. The terms or words used in the present specification and scope of claims are not to be interpreted in a limited sense to their common or dictionary meanings, but rather should be interpreted to the meanings and concepts that agree with the technical spirit of the present invention, based on the principle that the inventor may suitably define the concept of a term to best describe the invention. Also, technical terms and scientific terms used herein, unless defined otherwise, have the meanings understood by the person having ordinary skill in the field of art to which the present invention pertains. In the descriptions and appended drawings, descriptions of certain known functions and compositions are omitted, if it is deemed that such descriptions may unnecessarily obscure the essence of the present invention. The drawings briefly described in the following are provided as examples in order that the spirit of the present invention may be sufficiently conveyed to the skilled person. Therefore, the present invention is not limited to the drawings set forth below and can be realized in different forms. Throughout the specification, the same reference numerals represent the same components. It should be noted that, in the drawings, the same components are represented by the same numerals if possible, regardless of which drawing they are shown in.

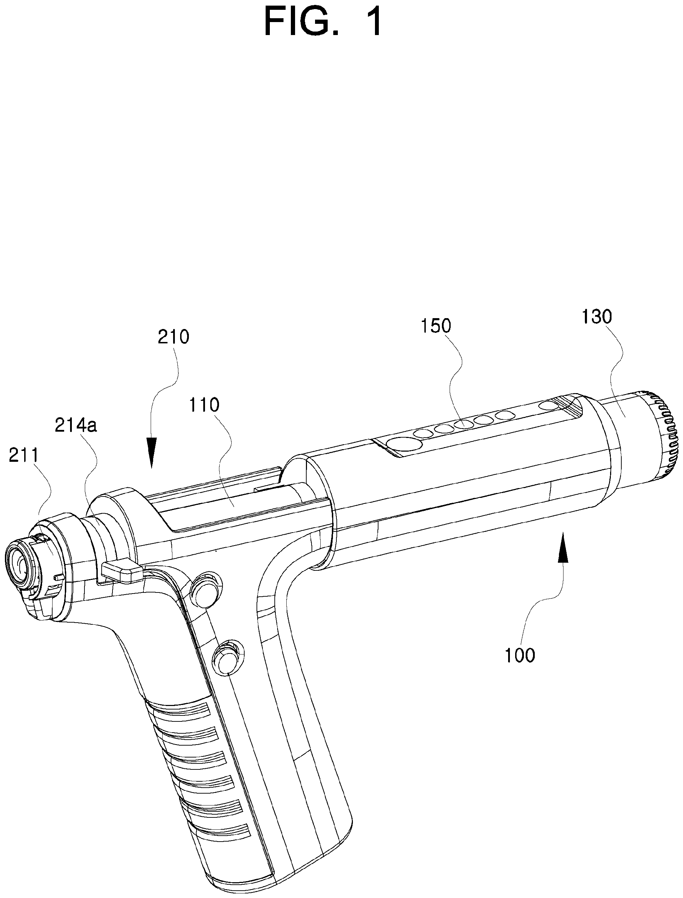

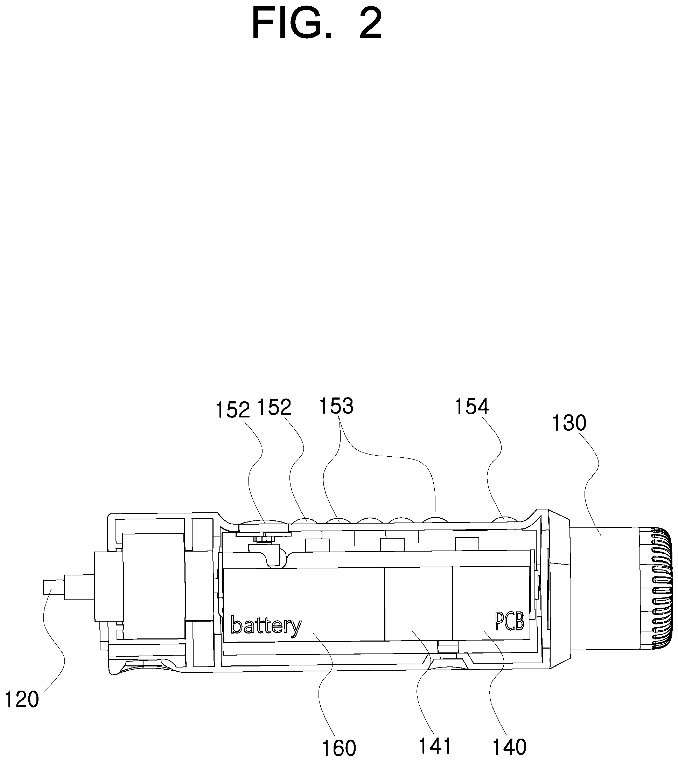

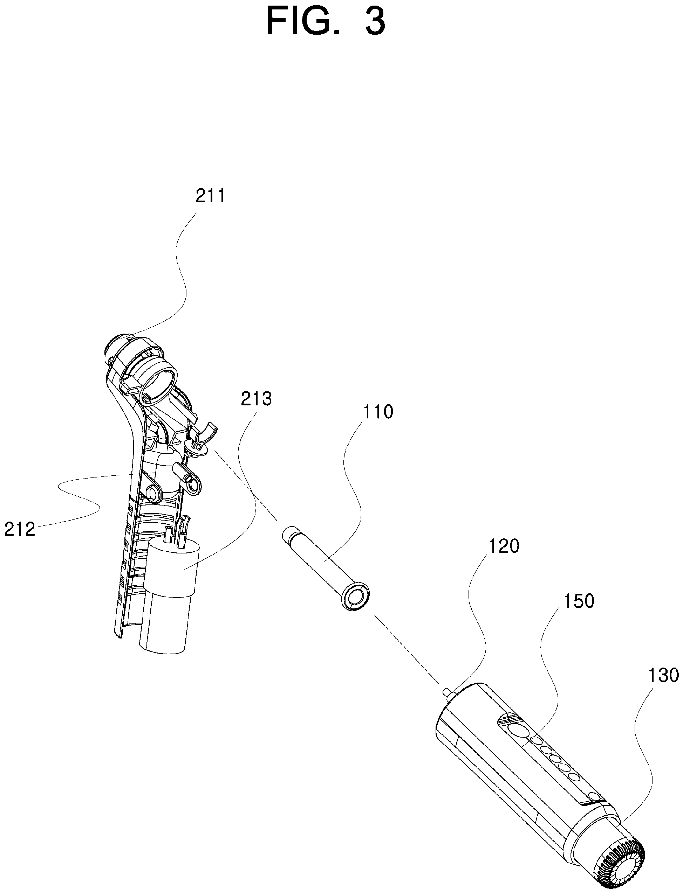

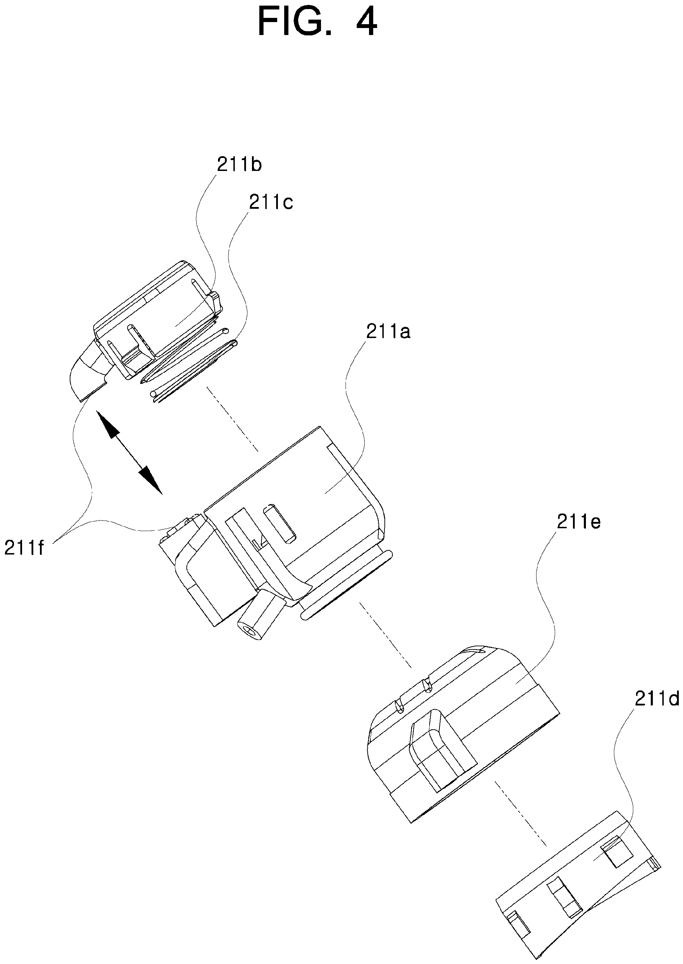

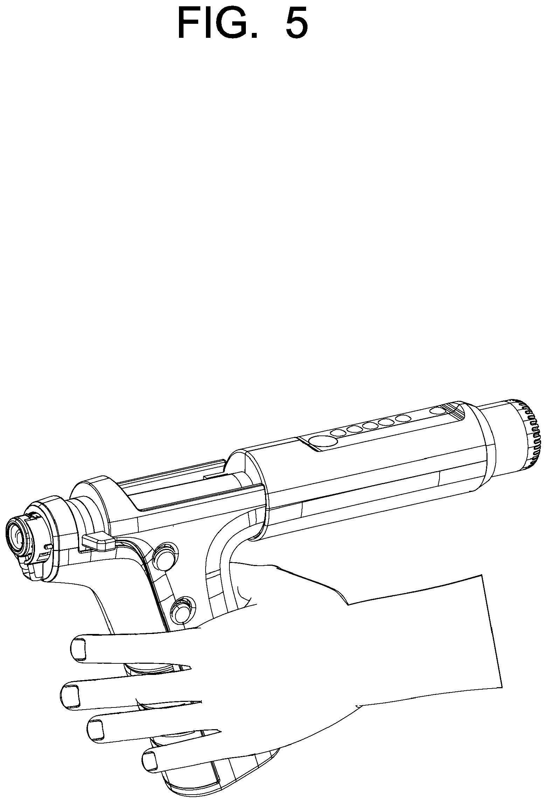

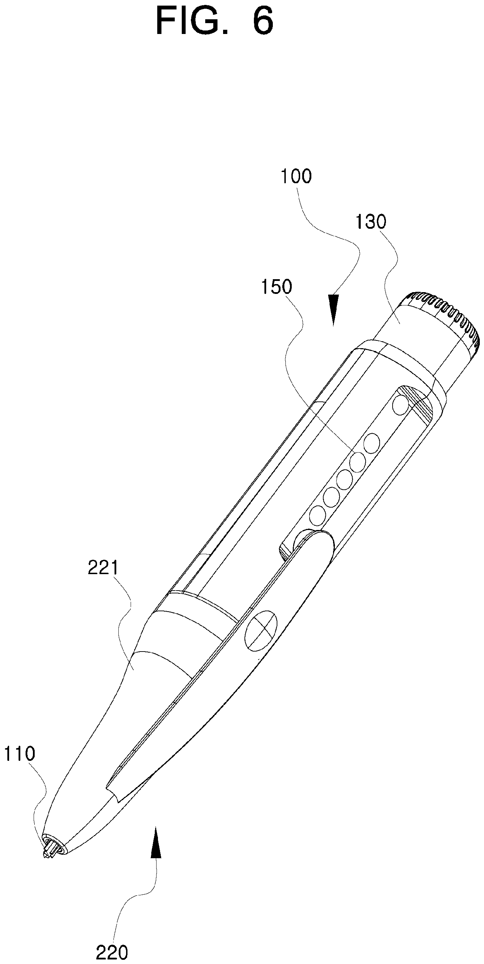

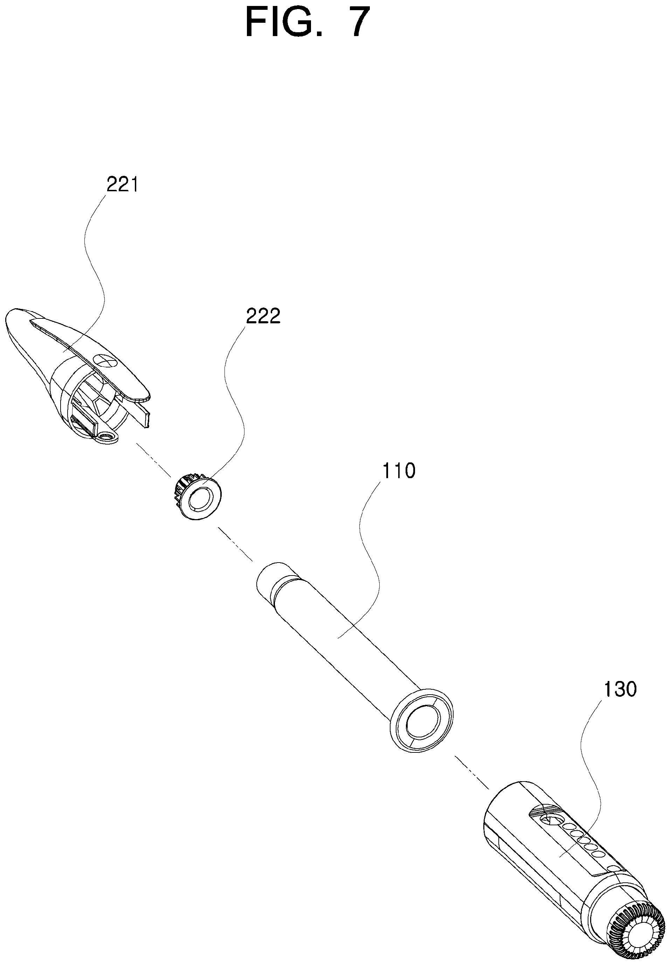

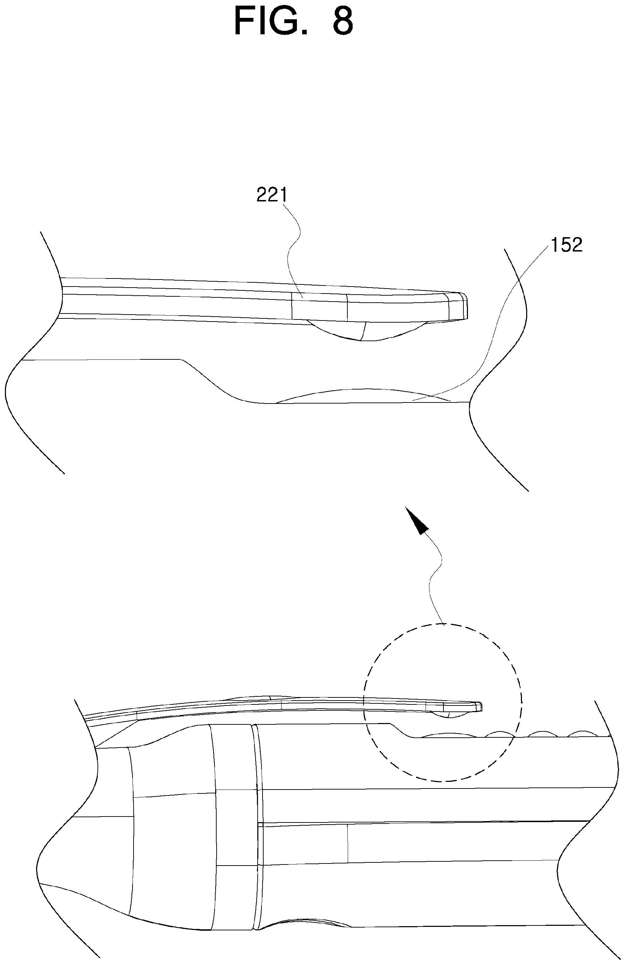

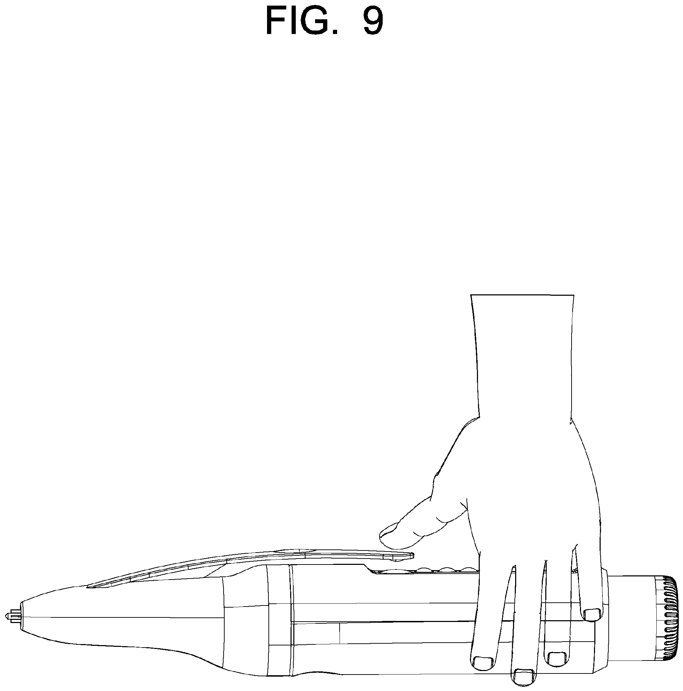

FIG. 1 shows an automatic multifunctional liquid injector having a separable applicator according to an embodiment of the present invention where a hydrolifting applicator unit is mounted, FIG. 2 shows a syringe actuation unit according to an embodiment of the present invention, FIG. 3 is an exploded perspective view of an automatic multifunctional liquid injector having a separable applicator according to an embodiment of the present invention where a hydrolifting applicator unit is mounted, FIG. 4 is an exploded perspective view of a suction tip according to an embodiment of the present invention, FIG. 5 shows an example of holding a hydrolifting applicator unit based on an embodiment of the present invention, FIG. 6 shows an automatic multifunctional liquid injector having a separable applicator according to an embodiment of the present invention where a filler applicator unit is mounted, FIG. 7 is an exploded perspective view of an automatic multifunctional liquid injector having a separable applicator according to an embodiment of the present invention where a filler applicator unit is mounted, FIG. 8 shows a syringe protection means 221 according to an embodiment of the present invention, and FIG. 9 shows an example of holding a filler applicator unit based on an embodiment of the present invention.

An automatic multifunctional liquid injector having a separable applicator conceived to resolve the problems described above, as illustrated in FIGs. 1 and 2, may include a syringe actuation unit 100, which includes a syringe 110, a syringe actuation shaft 120 coupled to a rear surface of the syringe 110 and configured to discharge a liquid inside the syringe 110 by way of a piston motion, a syringe actuation means 130 coupled with the syringe actuation shaft 120 to provide an actuation force for the piston motion of the syringe actuation shaft 120, a control means 140 configured to control the syringe actuation means 130, an input means 150 configured to input a command of a user into the control means 140, and a power supply means 160 configured to supply power to the syringe actuation means 130 and the control means 140; and an applicator unit 200 that is coupled in a replaceable manner to a front surface of the syringe actuation unit 100, wherein the applicator unit 200 may perform a hydrolifting procedure or a filler procedure.

That is, while using the syringe actuation unit 100 in the same manner, an applicator unit 200 for hydrolifting procedures or for filler procedures may be used selectively in performing each procedure. Thus, whereas in the past a device for hydrolifting procedures and a device for filler procedures had to be purchased separately for performing hydrolifting procedures and filler procedures, such procedures may all be performed with a single set of an automatic multifunctional liquid injector having a separable applicator based on the present invention. This allows increased economic efficiency in terms of purchasing equipment on the part of the cosmetic practitioner and also allows increased economic efficiency in terms of maintenance, as the costs associated with maintaining existing devices for hydrolifting procedures and filler procedures may be reduced.

The applicator unit 200 may further include a hydrolifting applicator unit 210, which in turn may include a suction tip 211 configured to directly contact a skin surface of the practitioner and suction waste matter, a waste storage chamber 212 configured to store the waste matter suctioned by way of the suction tip 211, and a suction means 213 configured to generate suction pressure at the suction tip 211.

That is, to perform a hydrolifting procedure, which involves suctioning waste matter from the skin surface by using a suctioning device and injecting a liquid from inside a syringe into the skin, the hydrolifting applicator unit 210 is used, which includes a suction tip 211 for suctioning waste matter via direct contact with the skin surface of the practitioner and suctioning the skin to facilitate the injection of the liquid from within the syringe 110, a waste storage chamber 212 for storing the waste matter suctioned through the suction tip 211, and a suction means 213 for generating a suction pressure at the suction tip 211.

Here, the suction tip 211 may include a suction tip body 211a, a skin contact part 211b that is coupled with the suction tip body 211a such that the skin contact part 211b may slide into the suction tip body 211a, an elastic element 211c that is disposed between the suction tip body 211 and the skin contact part 211b to provide an elastic force, and a syringe alignment part 211d that is coupled with the other side of the suction tip body 211a and configured to secure the position of the syringe 110.

As illustrated in FIG. 3, in an automatic multifunctional liquid injector having a separable applicator based on the present invention, the hydrolifting applicator unit 210 may include a syringe alignment part 211d in which the syringe 110 may be inserted and secured, so that the syringe 110 may be firmly secured to the hydrolifting applicator unit 210. Since the syringe alignment part 211d can secure the connection portion of the needle for syringes 110 of various diameters and thus can accommodate various diameters, the injector may be used in a wide range of applications. Here, the center of the syringe can be kept the same regardless of the diameter of the syringe secured to the syringe alignment part 211d, so that the position of the inserted needle remains unchanged. Therefore, the cosmetic practitioner may perform procedures more comfortably, and the level of precision may be improved as well.

Also, as illustrated in FIG. 4, the suction tip 211 based on the present invention has the skin contact part 211b, which is placed in direct contact with the skin of the person receiving the procedure, formed separately from the suction tip body 211a. Therefore, the cosmetic practitioner is able to replace the skin contact part 211b, which directly contacts the skin of the person receiving the procedure and thus requires meticulous care for hygiene, and conduct procedures in a hygienic manner. This may also provide an economic advantage, since just the skin contact part 211b may be replaced, in contrast to having to replace the entire suction tip for hygiene in existing equipment used in a hydrolifting procedure.

In addition, the suction tip 211 may further include a syringe position adjustment lever 211e that couples with the syringe alignment part 211d, where the coupling portions of the syringe position adjustment lever 211e and the syringe alignment part 211d may be tapered in a corresponding manner.

That is, as illustrated in FIG. 4, for the syringe position adjustment lever 211e and the syringe alignment part 211d that couple with each other, one side of the syringe alignment part 211d is tapered, and the syringe position adjustment lever 211e coupled to this is also tapered, so that the syringe position adjustment lever 211e is secured at the one end and the other end by way of a rotational movement. Using this, the syringe alignment part 211d may move forward and backward, to adjust the position of the syringe 110 and adjust the position of the inserted needle. In this way, it is possible to adjust the position of the inserted needle as necessary by moving the position of the syringe 110 secured to the syringe alignment part 211d, thereby improving the degree of freedom as well as convenience for the cosmetic practitioner when performing a procedure.

Moreover, the suction tip 211 may further include a contact operation switch 211f that is formed in corresponding positions of the suction tip body 211a and the skin contact part 211b and is configured to actuate the syringe actuation unit 100 and the suction means 213 by contact.

Thus, as illustrated in FIG. 5, when a cosmetic practitioner performs a procedure with the hydrolifting applicator unit 210 mounted, the practitioner may operate the automatic multifunctional liquid injector having a separable applicator of the present invention simply by contacting the suction tip 211 onto the skin, without the need for a separate operation switch, to perform a hydrolifting procedure. This provides the advantage of improved convenience for the cosmetic practitioner. Here, the elastic element 211c, which is provided between the suction tip body 211a and the skin contact part 211b where the contact operation switch 211f is implemented, may provide an elastic force between the suction tip body 211a and the skin contact part 211b, so that when the contact with the skin is disengaged, the skin contact part 211b may readily return to its original position. This may prevent possible malfunctions resulting from the suction tip body 211a and the skin contact part 211b failing to disengage contact. The elastic element 211c may also be implemented in a replaceable form, so that it may be replaced when the elasticity has been degraded, for maximized maintenance efficiency.

In addition, the suction tip 211 may be formed to have an inclination of 15 degrees with respect to the skin surface of the person receiving the procedure. It is known that, in a hydrolifting procedure, the optimal angle between the procedure equipment and the skin surface of the person receiving the procedure is 15 degrees. In existing equipment for hydrolifting procedures, the tip contacting the skin of the person receiving the procedure is formed in a straight shape, so that the cosmetic practitioner performing the procedure had to personally incline the equipment. However, with an automatic multifunctional liquid injector having a separable applicator according to the present invention, the suction tip 211 may be formed inclined at an angle of 15 degrees with respect to the skin surface of the person receiving the procedure and thus may be used at an optimal angle without having to be inclined by the cosmetic practitioner. This can improve the convenience of performing the procedure and can improve the efficiency of the procedure, since a cosmetic practitioner who is not highly skilled may still perform the procedure at an optimal angle.

Furthermore, the input means 150 may be positioned at an upper surface of the syringe actuation unit 100, and the applicator unit 200 may further include a filler applicator unit 220 that includes a syringe protection means 221, of which one side may be coupled to the syringe actuation unit 100 and which may be formed surrounding the syringe 110, and a syringe securing means 222, which may be formed between the syringe protection means 221 and the syringe 110 to secure the syringe 110.

That is, as illustrated in FIGs. 6 and 7, the syringe protection means 221 may prevent the syringe 110 from being directly exposed to the exterior and thus may prevent the syringe 110 from being damaged by external impact. As the syringe 110 is coupled with the syringe protection means 221 and the syringe securing means 222, the center of the syringe 110 is kept constant regardless of the diameter of the syringe 110, and the position of the inserted needle is kept constant as well. This may prevent accidents in the related art resulting from the syringe center changing positions depending on the diameter of the syringe and the need for the cosmetic practitioner to recognize such changes, and as such, the safety of the procedure may be increased regardless of the level of skill of the cosmetic practitioner.

In the filler applicator unit 220, as illustrated in FIG. 8, the syringe protection means 221 may have a portion of the upper surface extending to a position above the operation switch 152 of the input means 150, which may include a power switch 151, an operation switch 152, a mode change switch 153, and a data edit switch 154, and the syringe protection means 221 may have the portion for contacting the operation switch 152 of the input means 150 formed protruding downward. That is, in the case of a filler applicator unit 220, which is held in the manner illustrated in FIG. 9 and is operated by the cosmetic practitioner directly pressing the operation switch 152 of the input means 150 for use in a filler procedure, a portion of the upper surface of the syringe protection means 221 may extend to a point above the operation switch 152 of the input means 150, while the portion of the syringe protection means 221 intended for contact with the operation switch 152 may protrude downward, so as to more easily contact the operation switch 152 when the cosmetic practitioner presses the operation switch 152 for the procedure. In this way, the automatic multifunctional liquid injector having a separable applicator based on the present invention may maximize the procedure convenience of the cosmetic practitioner by allowing the cosmetic practitioner to perform the procedure with a comfortable grip.

Furthermore, the syringe actuation unit 100 may include a mode storage device 141, on which a plurality of operation modes is recorded, where each operation mode may be configured to enter an injection speed and an injection amount set beforehand into the control means 140 and stop automatically.

The control values for the syringe actuation means 130 of the syringe actuation unit 100 for controlling the injection speed and injection amount with which the liquid inside the syringe 110 is injected into the skin should vary according to the conditions of each procedure, including the type of procedure, such as a hydrolifting procedure and a filler procedure, and the type of syringe 110. As such, in the related art, the cosmetic practitioner would respond to such conditions by directly manipulating the injection speed and injection amount based on personal experience. This method is vulnerable to error-induced accidents even with highly skilled cosmetic practitioners, and there is also the problem that the differences in outcome recognized by the patient vary greatly according to the level of skill of the cosmetic practitioner.

An automatic multifunctional liquid injector having a separable applicator based on the present invention may respond to the different procedure conditions by performing the injection operation with the unique injection speed and unique level set beforehand for one of the plurality of operation modes recorded on the mode storage device 141 and stopping automatically afterwards. That is, the cosmetic practitioner determines the procedure conditions and, through the input means 150, selects an operation mode matching the procedure conditions from among the plurality of operation modes recorded on the mode storage device 141 to perform the procedure. In this way, the automatic multifunctional liquid injector having a separable applicator according to the present invention may help the cosmetic practitioner perform a procedure more conveniently by providing an optimal operation mode for a variety of procedure conditions.

Also, the control means 140 may further include a manual control mode, in which the injection operation may be performed continuously for as long as the operation switch 152 of the input means 150 is pressed.

That is, if a cosmetic practitioner using an automatic multifunctional liquid injector having a separable applicator based on the present invention does not wish to control the injection of the liquid within the syringe 110 to follow a control mode, the cosmetic practitioner may select the manual control mode and personally control the syringe actuation unit 100, to control the speed and amount in which the liquid from inside the syringe 110 may be injected. This may improve convenience, as the procedure may be performed according to the intentions of the skilled cosmetic practitioner.

While the present invention is described above by way of details such as specific components and drawings relating to limited embodiments, these are provided merely to aid the overall understanding of the present invention. The present invention is not limited to the embodiments set forth above, and the person having ordinary skill in the field of art to which the present invention pertains would be able to derive numerous modifications and variations.

As such, the spirit of the present invention is not to be confined to the embodiments described above, but rather the scope of claims set forth below as well as all variations of a substitutive or equivalent nature are encompassed within the scope of the present inventive spirit.

## Claims

1. An automatic multifunctional liquid injector having a separable applicator, the injector comprising:
a syringe actuation unit (100) comprising a syringe (110), a syringe actuation shaft (120) coupled to a rear surface of the syringe (110) and configured to discharge a liquid inside the syringe (110) by way of a piston motion, a syringe actuation means (130) coupled with the syringe actuation shaft (120) to provide an actuation force for the piston motion of the syringe actuation shaft (120), a control means (140) configured to control the syringe actuation means (130), an input means (150) configured to input a command of a user into the control means (140), and a power supply means (160) configured to supply power to the syringe actuation means (130) and the control means (140); and
an applicator unit (200) coupled in a replaceable manner to a front surface of the syringe actuation unit (100),
wherein the applicator unit (200) performs a hydrolifting procedure or a filler procedure.

2. The automatic multifunctional liquid injector having a separable applicator according to claim 1, wherein the applicator unit (200) further comprises a hydrolifting applicator unit (210),
the hydrolifting applicator unit (210) comprising a suction tip (211) configured to directly contact a skin surface of a practitioner and suction waste matter, a waste storage chamber (212) configured to store the waste matter suctioned by way of the suction tip (211), and a suction means (213) configured to generate suction pressure at the suction tip (211).

3. The automatic multifunctional liquid injector having a separable applicator according to claim 2, wherein the suction tip (211) comprises:
a suction tip body (211a), a skin contact part (211b) coupled with the suction tip body (211a) such that the skin contact part (211b) slides inside the suction tip body (211a), an elastic element (211c) disposed between the suction tip body (211) and the skin contact part (211b) to provide an elastic force, and a syringe alignment part (211d) coupled with the other side of the suction tip body (211a) and configured to secure a position of the syringe (110).

4. The automatic multifunctional liquid injector having a separable applicator according to claim 3, wherein the suction tip (211) further comprises a syringe position adjustment lever (211e) coupled with the syringe alignment part (211d),
wherein coupling portions of the syringe position adjustment lever (211e) and the syringe alignment part (211d) are tapered in a corresponding manner such that the syringe position adjustment lever (211e) is secured at one end and the other end by way of a rotational movement.

5. The automatic multifunctional liquid injector having a separable applicator according to claim 3, wherein the suction tip (211) further comprises:
a contact operation switch (211f) formed in corresponding positions of the suction tip body (211a) and the skin contact part (211b), the contact operation switch (211f) configured to actuate the syringe actuation unit (100) and the suction means (213) by way of contact.

6. The automatic multifunctional liquid injector having a separable applicator according to claim 2, wherein the suction tip (211) is formed at an angle of 15 degrees with respect to a skin surface of a person receiving a procedure.

7. The automatic multifunctional liquid injector having a separable applicator according to claim 1, wherein the input means (150) includes a power switch (151), an operation switch (152), a mode change switch (153), and a data edit switch (154).

8. The automatic multifunctional liquid injector having a separable applicator according to claim 7, wherein the input means (150) is positioned at an upper surface of the syringe actuation unit (100), and
the applicator unit (200) further comprises a filler applicator unit (220),
the filler applicator unit (220) comprising a syringe protection means (221) having one side thereof coupled to the syringe actuation unit (100) and formed surrounding the syringe (110), and a syringe securing means (222) formed between the syringe protection means (221) and the syringe (110) to secure the syringe (110).

9. The automatic multifunctional liquid injector having a separable applicator according to claim 8, wherein the syringe protection means (221) has a portion of an upper surface thereof extending to a position above the operation switch (152) of the input means (150), and a portion for contacting the operation switch (152) of the input means (150) is formed protruding downward.

10. The automatic multifunctional liquid injector having a separable applicator according to claim 1, wherein the syringe actuation unit (100) comprises a mode storage device (141),
the mode storage device (141) having a plurality of operation modes recorded thereon, each operation mode configured to enter an injection speed and an injection amount set beforehand into the control means (140) and stop automatically.

11. The automatic multifunctional liquid injector having a separable applicator according to claim 10, wherein the control means (140) further includes a manual control mode,
the manual control mode configured to perform an injection operation as long as an operation switch (152) of the input means (150) is pressed.
